# EUROPEAN PATENT SPECIFICATION

(11) **EP 0 487 229 B1**
(45) Date of publication and mention of the grant of the patent: **24.09.1997**
(21) Application number: 91310334.7
(22) Date of filing: 07.11.1991
(51) Int. Cl.: C12N 15/12, C07K 14/435

(54) **A DNA encoding a protein which binds to the enhancer of alpha-fetoprotein gene**
Für ein Protein, das sich mit dem Enhancer des Alpha-Fetoprotein verbindet, kodierende DNS
DNA codant pour une protéine se liant au gène de l'agent de renforcement de l'alpha fetoprotéine

(30) Priority: 07.11.1990 JP 301412/90
(43) Date of publication of application: 27.05.1992
(73) Proprietor: SNOW BRAND MILK PRODUCTS CO., LTD., Sapporo-shi, Hokkaido 065 (JP)
(72) Inventor: Morinaga, Tomonori 3A, Kurai Manshon,, Tochigi-ken (JP); Yasuda, Naotaka 2A, Okada Manshon,, Tochigi-Ken (JP); Higashio, Kanji, Saitama-Ken (JP); Tamaoki, Taiki, Calgary Alberta (CA)
(74) Representative: Davies, Jonathan Mark

(56) References cited:
- EP-A- 0 427 863
- MOLECULAR AND CELLULAR BIOLOGY vol. 8, no. 12, December 1988, WASHINGTON, DC USA pages 5179 - 5187; Sawadaishi, K. et al.: 'Interaction of a Hepatoma- Specific Nuclear factor with transcription-regulatory sequences of the HumanAlpha-Fetoprotein and albumin genes.'
- BIOCHEMICAL AND BIOPHYSICAL RESEARCH COMMUNICATIONS. vol. 143, no. 1, 27
- February 1987, DULUTH, MINNESOTA US Wang, Zhi; Cote, Gilbert J.; Chiu, Jen Fu: 'A specific .alpha.-fetoprotein gene binding protein in .alpha.-fetoprotein producing rat hepatomas'
- JOURNAL OF BIOLOGICAL CHEMISTRY. vol. 262, no. 10, 5 April 1987, BALTIMORE US pages 4812 - 4818; WATANABE, K.ET AL.: 'Cell-specific enhancer activity in a far upstream region of the human alpha fetoprotein gene'
- Cell (1989) 59:145

## Description

The present invention concerns a DNA sequence encoding a protein which specifically binds to the enhancer of α-fetoprotein gene and enhances the transcription of α-fetoprotein gene.

Expression of the genetic information in eukaryotes is regulated at the steps of transcription, RNA processing, translation and processing after the translation. Of all the regulation steps, that at the transcription level gives the stronger effect on the expression of the genetic information.

In the 5'-flanking region of genes which are transcribed by RNA polymerase II a promoter and, in some cases, enhancers which are involved in the transcription of the gene are present.

Recently, nuclear factors which bind to promoters or enhancers by recognizing the specific nucleotide sequences in the promoter or the enhancer were found. And it was found that the acitivity of promoter was regulated by the binding of these nuclear factors. The proteins which enhance transcription by RNA polymerase by binding to TATA box, CAAT box or GC box have been studied and the cDNAs for these proteins have been cloned.

The studies on the factors that bind to enhancers are also in progress. For example, cDNAs of OTF-2 (Octamer transcription factor-2) [Michael et al., Nature 336, 544-551, (1988)], which is B-cell specific and binds to the enhancer of immunogloblin κ-chain gene, and OTF-1, which recognizes the same nucleotide sequence and present ubiquitously in many tissues, have been isolated.

In the human α-fetoprotein gene, the presence of a region having enhancer activity at 3.5 kb upstream of the promoter was found.

A factor which binds to this enhancer was found and was termed AFP-1 [Sawadaishi et al., (Molecular and Cellular Biology), vol.8, 5179-5187(1988)]. However, the protein structure or the physicochemical characteristics of AFP-1 is unknown and the gene encoding AFP-1 has not been isolated.

In the investigation of the nuclear factor which specifically binds to the region having TTAATAATTA core sequence present in the enhancer of the human α-fetoprotein gene, the inventors have isolated a cDNA encoding the factor and determined the nucleotide sequence of the cDNA.

Frain et al, described a DNA-binding protein termed LF-B1 which they purified from rat river nuclei [Cell 59, 145-157 (1989)]. LF-B1 binds AT-rich sequences in the promoters of many liver-specific genes and activates their expression. The complete nuclueotide sequence of the cDNA for rat LF-B1 was determined and the amino acid sequence was deduced. Although LF-B1 binds the nucleotide sequences that resemble the target sequence for the DNA-binding protein described in the present application, these two proteins are distinct from each other.

A portion of the cDNA for the enhancer-binding protein described in the present application has been described in a European patent application, EP A 0 473 863, unpublished at the priority date of this application.

The present invention offers a novel DNA sequence which codes for the protein that specifically binds to the enhancer of the human α - fetoprotein gene. This DNA may be useful for the production of biologically active proteins by inserting this DNA into an expression vector and co-transfecting animal cells with another expression vector in which a gene for the biologically active protein is placed under the control of α - fetoprotein gene enhancer and promoter.

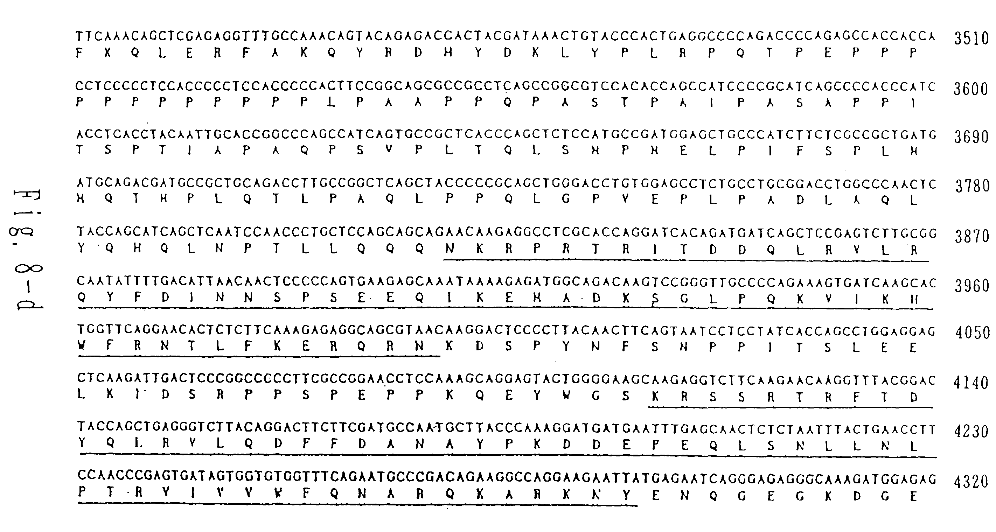

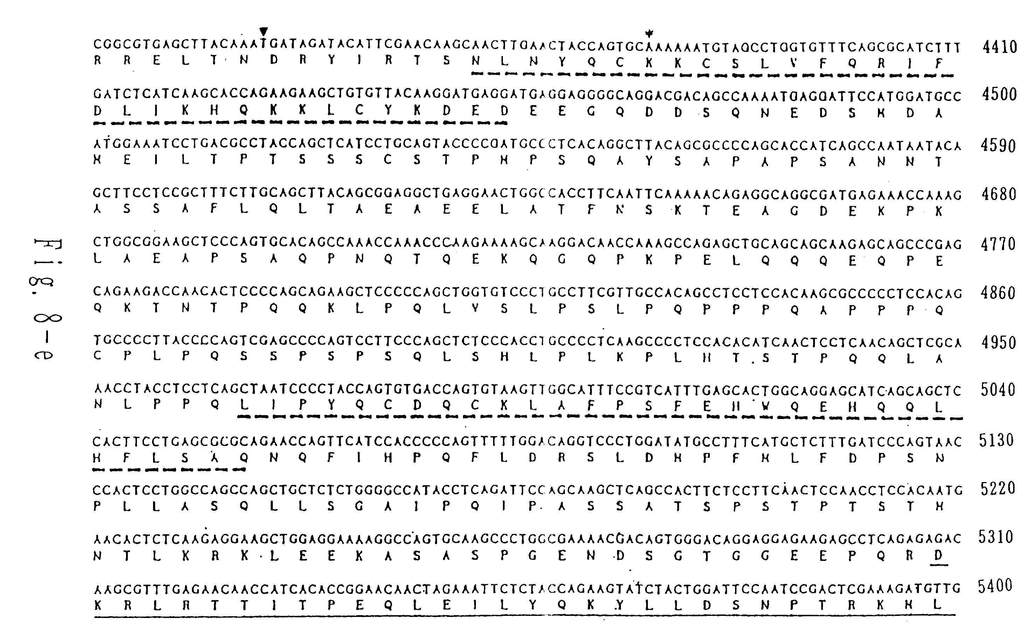

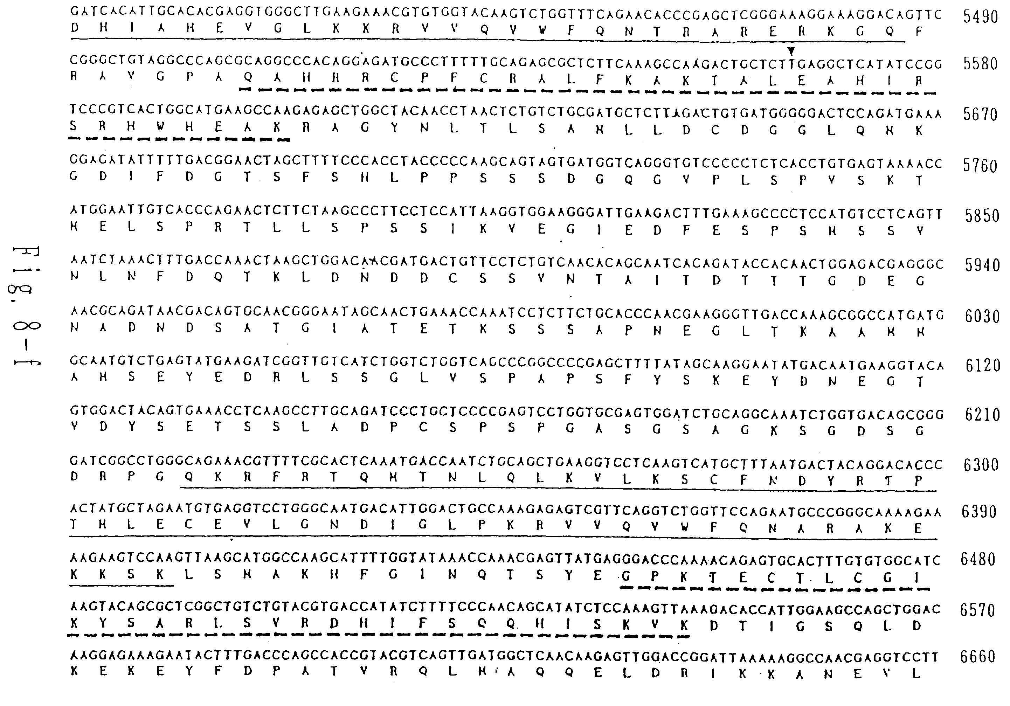

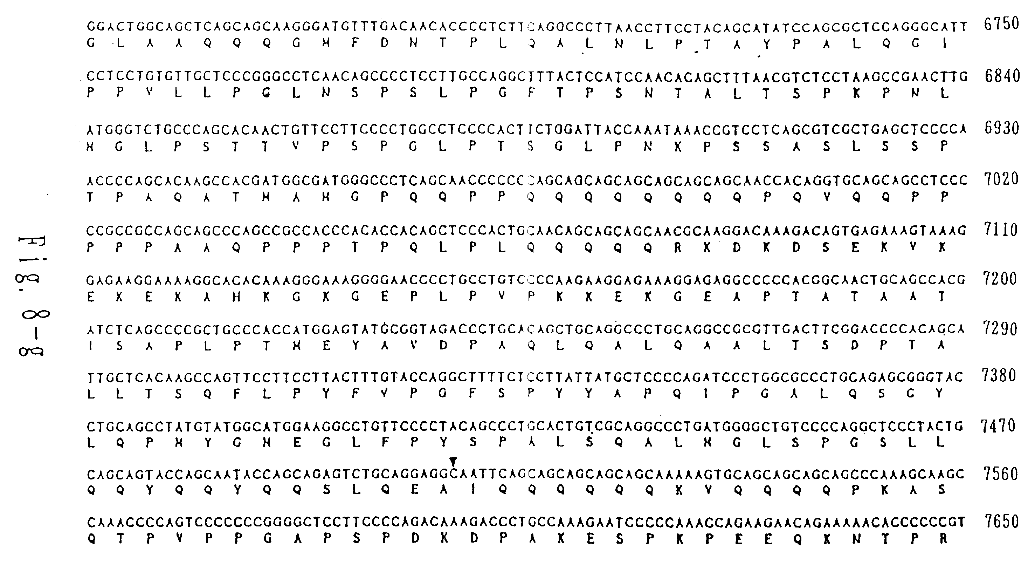

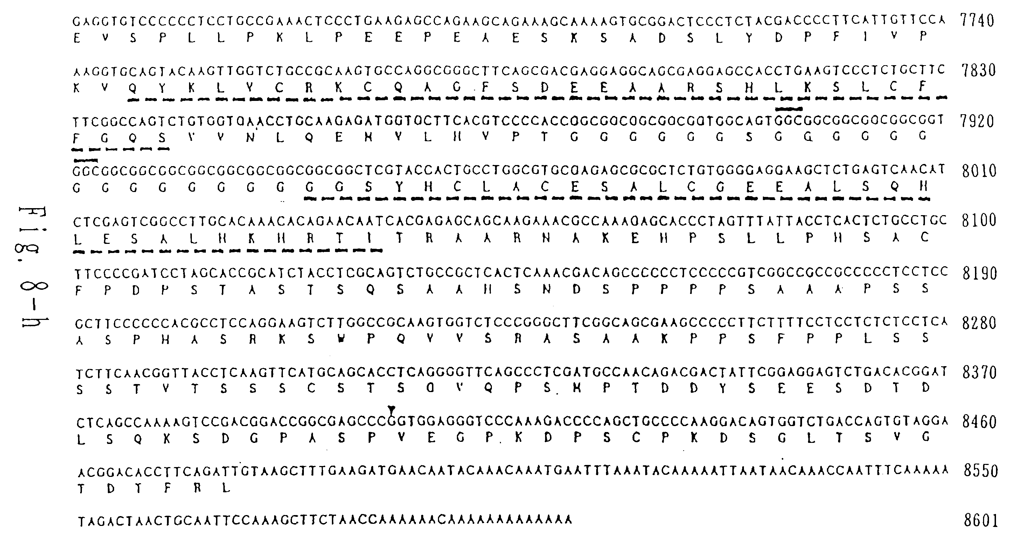

The present invention concerns a DNA having a nucleotide sequence shown in Fig 8. and encoding a protein which binds specifically to the human α - fetoprotein gene enhancer. The nucleotide sequence shown in Fig 8. is an example of such DNA sequence. This invention includes such DNAs which contain point mutations from which replacement of amino acid residues could result. An example of such mutation is the mutation at nucleotide 7508 in the sequence shown in Fig. 8. This mutation resulted in a replaced amino acid residue.

Fig.1 shows restriction enzyme map of the enhancer region present in the 5'-flanking region of the human α-fetoprotein gene. The region from 3.3kb to 3.7kb upstream of the transcription intiation site is presented magnifying the scale. Hi and Ha represent recognition sites for restriction enzymes Hind III and Hae III, respectively.

Fig.2 shows construction of CAT plasmid pAF1.OE25 CAT. A plasmid pAF1.OE25CAT is constructed by inserting NIa IV/Xho I fragment (31bp) of the enhancer region into the Cla I site of plasmid pAF1.OCAT.

Fig.3 shows the result of a CAT assay using HuH-7 human hepatoma cells. As shown, NIa IV/Xho I fragment has an enhancer activity.

Fig.4 shows the procedure to clone cDNA synthesized from mRNA into λ gt11 expression vector. A vertical arrow in the figure indicates the site in the lacZ gene to where cDNA is inserted.

Fig.5 shows restriction map of the cDNA and positions of partial cDNA clones.

Fig.6 shows the results of gel mobility shift assay with extracts prepared from λ2 and λ gt11 lysogens. Control indicates an assay without lysogen extract. Two shifted bands (a and b) appear only when extract prepared from λ2 lysogen is used.

Fig.7 shows the result of DNase I footprint analysis with λ2 lysogen extract. G + A represents a size marker which was prepared by the chemical cleavage of the probe DNA according to the method of Maxam and Gilbert. DNA extracted from the band which was not shifted was run in lane 1. Lanes 2 and 3 show DNA extracted from shifted bands a and b, respectively. Panel A shows the result obtained by labelling the 5'-terminus of Sma I site. Panel B shows the pattern obtained by labelling the 5'-terminus of Hinc II site.

Fig.8 shows the nucleotide sequence of AFP-1 cDNA and amino acid sequence deduced from it. Nucleotides that differ between two overlapping clones are indicated by closed triangles (▼). Asterisk (∗) indicates adenine missing in λ 488. Homeodomains are indicated by solid underlines and zinc-fingers are indicated by broken underlines.

The protein described in their invention binds to the enhancer of human α-fetoprotein gene and has a primary amino acid sequence shown in Fig.8.

One can prepare such DNA from mRNA extracted from the α-fetoprotein-producing cells of hepatocyte lineage. HuH7, HepG2 and Hep3B are the examples of such cell which produce α-fetoprotein.

In the present invention, cells producing α-fetoprotein were cultured and RNA was extracted from the cells. Poly(A) RNA was prepared from the RNA by means of oligo(dT) cellulose chromatography according to the standard procedure.

An example of the procedure to be used to extract RNA from cultured cells is guanidinium isothiocyanate-cesium chloride procedure [J.M. chirgwin et al., (Biochemistry 18, 5294 (1979))].

cDNA was synthesized using poly(A) RNA collected as mentioned above as template for reverse transcryptase. After the formation of double stranded cDNA from the single stranded cDNA. The product was ligated to λgt11 DNA and recombinant phage was constructed by packaging the recombinant DNA into λ -phage. Commercially available packaging system, packagene packaging system, for example (Promega), can be used for λ -phage packaging.

Then, recombinant phage prepared by the procedure mentioned above was infected to E.coli, for example to E.coli Y 1090(r⁻), and proliferated on agarose plates to obtain cDNA library. Screening was carried out by using DNA of enhancer region of α-fetoprotein gene as a probe.

DNA coding for the protein which binds to the enhancer of α-fetoprotein gene could be obtained.

The following is a detailed description of the invention.

### Example

This example shows the preparation of DNA coding for a protein that binds to the enhancer of α-fetoprotein gene from mRNA prepared from human hepatoma-derived cell line, HuH-7.

### ① Confirmation that the DNA probe having enhancer activity:

The fact that the enhancer activity resides on the DNA which was used as a screening probe was confirmed by the following CAT assay.

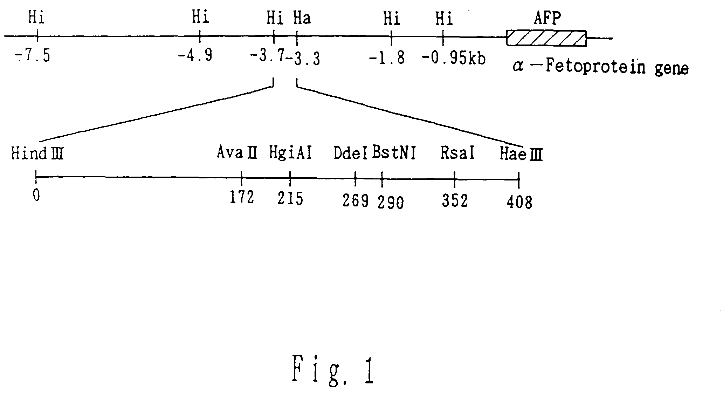

The DNA fragment having enhancer activity [(refer to Fig.1 and Molecular and Cellular Biology 8, 5179 (1988)], was isolated from the human genomic DNA by the digestion with restriction enzymes HgiA I and BstN I. The HgiA I/BstNI fragment was purified by agarose gel electrophoresis, and its termini were filled in with DNA polymerase I Klenow fragment. pUC18 plasmid was digested with BamH I and the ends were treated in the same manner. The two fragments were ligated using T4DNA ligase (Takara Shuzo Co., Ltd.) to give recombinant plasmid pAFE (HgiA I/BstN I)₁.

The plasmid pAFE(HgiA I /BstN I)₁ was subsequently digested with restriction enzymes Xba I and Pst I, and nucleotides were deleted from the ends with exonuclease III and mung-bean nuclease (Takara shuzo). Then the ends were filled in with DNA polymerase I Klenow fragment and the plasmid was recircularized after ligating Xho I linkers to the ends.

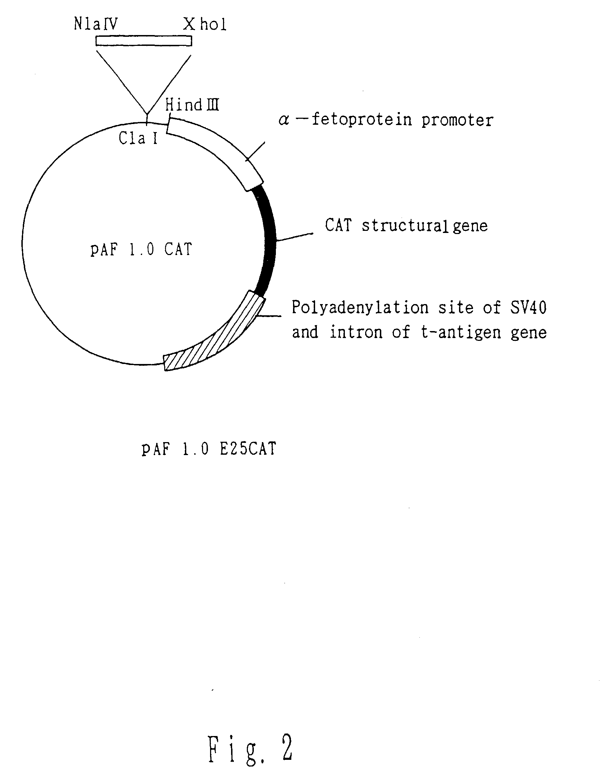

E. coli DH5 was transformed with the recircularized plasmid and a clone which underwent deletion down to nine bases downstream from TTAATAAT sequence was isolated. This plasmid was designated as pAFE (HgiA I /Xho I)₁. The plasmid pAFE(HgiA I /Xho I)1 was then hydrolyzed with restriction enzymes N1aIV and Xho I, and the fragment containing TTAATAA sequence was isolated by agarose gel electrophoresis. Ends of the Nla IV/Xho I fragment were filled in with DNA polymerase I Klenow fragment, and then the Nla IV/Xho I fragment was inserted into the Cla I site of plasmid pAF1.OCAT. The obtained plasmid was designated as plasmid pAF1.OE25CAT(see Fig.2).

The plasmid pAF1.0 CAT contains one kb DNA of the α -fetoprotein gene promotor region, structural gene for chloramphenicol acetyltransferase (CAT) of E.coli, SV40 poly(A) addition signal and t-antigen intron. The vector is used to screen for DNA fragment having enhancer activity [J. Biol.Chem., 262, 4812 (1987)]. Transfection of CAT plasmids into HuH-7 cells and CAT assay were performed as follows, based on the known method [Mol. Cell Biol., 2 , 1044(1982)].

About 8×10⁶ cells were grown in a 75 cm² culture flask. To these cells, 20 to 30 µg CAT plasmid DNA was transfected by calcium phosphate method. The cells were incubated for two days in RPMI-1640 culture medium containing 0.3% lactalbumin hydrolysate, after which the cells were washed with PBS, and scraped off using a scraper. Then the cells were collected by low-speed centrifugation (800 rpm, 5min.), washed once with a solution composed of 40mM Tris-HCl(pH7.5), one mM EDTA and 150mM NaCl, and suspended in 100µl of 250 mM Tris-HCl (pH7.5). This suspension was repeatedly frozen and thawed five times to lyse the cells, and centrifuged in an Eppendorf centriguge at 12,000rpm for five minutes to obtain a supernatant, which is then heat-treated at 65 °C for 10 minutes. The treated supernatant was again centrifuged at 12,000rpm for five minutes with the same machine to obtain a supernatant which was used as a cell extract.

After determining the protein concentration of the cell extract using the protein assay kit (Biolad), 50-200 µg of protein was used for the following CAT assay.

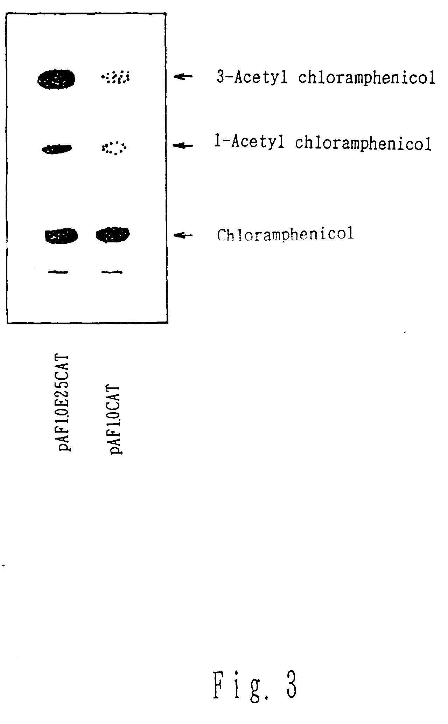

A typical CAT assay reaction mixture, 180 µl, contains 250mM Tris-HCl (pH 7.5), 0.1 µ Ci[¹⁴C]chloramphenicol (Amersham), 0.4mM acetyl-CoA, and 50-200 µg protein extracted from the cells. This reaction mixture was incubated at 37°C for 60-180 minutes, after which one ml of ethyl acetate was added to extract chloramphenicol and its acetylated derivatives. The ethylacetate layer was then separated and dried in vacuo. The residue was redissolved in 20 µl of ethyl acetate, a portion or all of which was applied onto a sillica gel plate (Merk). The resultant plate was then developed using chloroform:
methanol (96:4. V/V). The silica gel plate was dried at room temperature, wrapped with wrapping film, and subjected to autoradiography. As illustrated in Fig.3, a greater CAT activity was observed when the experiment was performed with CAT plasmid, pAF1.OE25CAT, which contained Nla IV/Xho I fragment derived from α-fetoprotein gene enhancer, than with pAF1.OCAT, indicating the presence of an enhancer activity in the Nla IV/Xho I fragment.

### ② Growth of α-fetoprotein producing cells:

Human hepatoma line, HuH-7, which produces α-fetoprotein was cultured under the following conditions. The cell line is available from Professor Jiro Sato, Pathology Division, Cancer Institute of Okayama University, Japan.

### Conditions for culture:

The medium used was RPMI-1640 medium supplemented with 0.3% (W/W) lactalbumin hydrolysate (Gibco) or RPMI-1640 medium containing 5-10% (V/V) fetal calf serum. The medium was replaced as needed, and incubation was performed in an incubator (37°C) filled with air containing 5% carbon dioxide.

### ③ Preparation of poly (A) RNA from the cells described above:

Total RNA was extracted from 2×10⁸ cells, adopting guanidinium isothiocyanate-cesium chloride method [Biochemistry, 18, 5294(1979)], by the following procedure.

20ml solution composed of sixM guanidirium isothiocyanate, five mM sodium citrate, 0.1M 2-mercaptoethanol and 0.5% sodium N-lauroyl sarcosinate was added to the cells, and the cells were homogenized at room temperature, then four g of cesium chloride was dissolved per 10ml of this homogenate. Over the cushion of 2.5 ml solution composed of 5.7 M cesium chloride and 0.1M EDTA (pH7.5), in a polyallomer tube, 10 ml of the homogenate was layered. The tube was then centrifuged at 34,000rpm for 18 hours at 20°C in Hitachi Ultracentrifuge Rotor RPS 40T (Hitachi Ltd.). The resultant precipitate was dissolved in one ml solution composed of 10mM Tris-HCl (pH 7.4), 5 mM EDTA and 1% SDS. To this solution, an equal volume of a mixture of chloroform and n-butanol (4:1, V/V) was added, mixed well and the mixture was centrifuged (16,000g, 10min.). To the obtained aqueous phase, one-tenth volume of 3M sodium acetate (pH 5.5) and 2.5 volumes of ethanol were added and mixed well. The mixture was allowed to stand at -70°C for more than two hours and centrifuged (16,000g 20min.) to precipitate RNA. The precipitate was washed with 70% ethanol and then dried (total RNA).

For the preparation of poly(A) RNA from the total RNA, oligo(dT) cellulose chromatography was employed as described below. Oligo (dT) cellulose (50mg) was packed in a small column and equilibrated with a solution composed of 10 mM Tris-HCl (pH 7.5), 0.5M NaCl, one mM EDTA and 0.1% SDS. Then 390 µg of the total RNA dissolved in the same buffer was applied on the column. RNA that was not retained was washed out by the buffer, and the retained RNA was eluted with a solution composed of 10mM Tris-HCl(pH7.5), one mM EDTA and 0.05% SDS. To this eluate, 1/10 volume of 3M sodium acetate (pH5.5) and 2.5 volumes of ethanol were added, mixed, and the mixture was allowed to stand for more than two hours at -70 °C. The mixture was then centrifuged (12,000g 15min.). The precipitate formed was washed with 70% ethanol, dried and dissolved in sterilized distilled water. This fraction was designated as poly(A) RNA.

### ④ Synthesis of cDNA:

cDNA synthesis kits purchased from Pharmacia and Amersham were used for this purpose.
(i) cDNA synthesis using random primer
   To the first-strand synthesis reaction mixture (32 µl), 2.5 µg of poly(A) RNA and 0.4 µg of random primer, dp(N)6 (Takara Shuzo Co., Ltd.) were added and then allowed to react according to the supplier's protocol(Pharmacia). An EcoR I adaptor was ligated to both ends of double-strand cDNA as directed by the protocol. From 2.5 µg poly (A) RNA, 2.3 µg of doublestranded cDNA was obtained.
(ii) cDNA synthesis using oligo (dT) primer
   One µg of double-strand cDNA was synthesized from 2. 5 µg of poly(A)RNA according to the protocol provided by Amersham. EcoR I adaptor was ligated to the ends of double-strand cDNA as directed by the protocol. Finally, 200ng of double-strand DNA with adaptor was obtained.

### ⑤ Preparation of recombinant DNA and recombinant phages:

Protoclone λ gt11 system (Promega) was used for the preparation of recombinant DNA. Ligation was performed according to the protocol. The recombinant DNA thus obtained was packaged into recombinant phage using Packer Gene Packaging System (Promega).

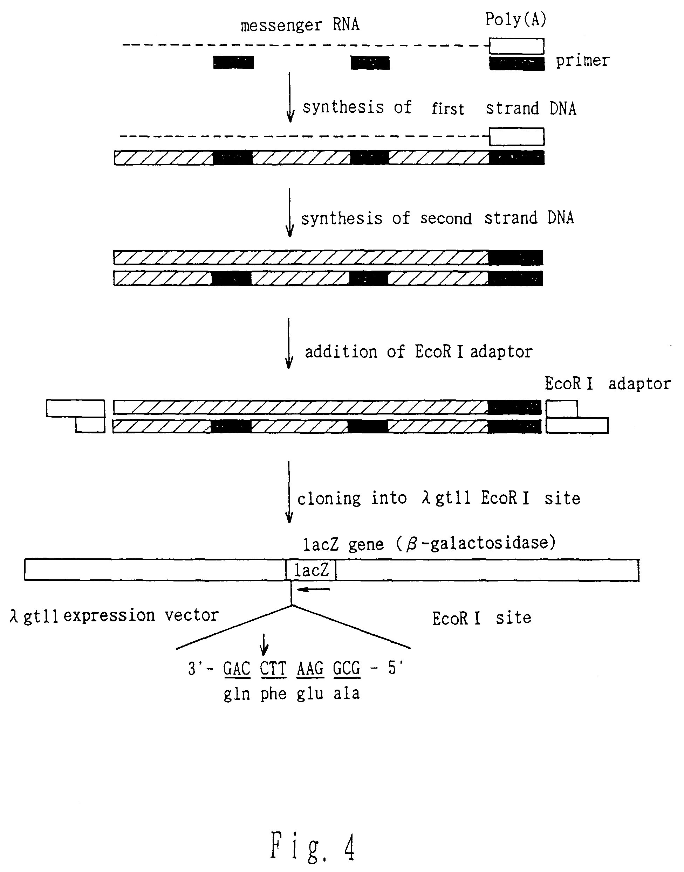

The process of preparing cDNA and λ gt11 recombinant phage is shown in Fig.4.

### ⑥ Preparation of λ gt11 cDNA library:

The recombinant phage packaged as described above was infected to E.coli Y1090(r⁻) and λ gt11 cDNA library was prepared according to the known method [DNA cloning, Vol.1, p49, IRL Press (1985)].

### ⑦ Preparation of probes and the screening method:

The probe for the screening was prepared as follows from the DNA fragment of the human α-fetoprotein gene ephancer located upstream of the gene (see Fig.4).

The DNA fragment having enhancer activity [described in Molecular and Cellular Biology 8, 5179 (1988)], was isolated by digesting genomic DNA with restriction enzymes HgiA I and BstN I. HgiA I/BstN I fragment was purified by agarose gel electrophoresis, and its termini were filled in with DNA polymerase I Klenow fragment. pUC18 plasmid was treated with BamH I and the ends were similarly treated. The two fragments were ligated using T4 DNA ligase (Takara Shuzo Co., Ltd.) to give a recombinant plasmid pAFE (HgiA I/BstN I)¹.

The pAFE(HgiA I/BstN )¹ was subsequently digested with restriction enzymes Xba I and Pst I and nucleotides were deleted from the ends with exonuclease III and mung-bean nuclease. Ends were filled in with DNA polymerase I Klenow fragment and the plasmid was recircularized by inserting Xho I linker to the ends.

A clone which underwent deletion down to nine bases downstream from TTAATAAT sequence was isolated from the deletion mutants obtained by transforming E. coli DH5 with the plasmid and designated as pAFE(HgiA I/Xho I)1.

The plasmid pAFE(HgiA I /Xho I)1 was then hydrolyzed with restriction enzymes Nla IV and Xho I , and a fragment containing the enhancer was isolated by agarose gel electrophoresis.

Ends of the Nla IV/Xho I fragment were filled in with DNA polymerase I Klenow fragment and the fragment was self-ligated using T4 DNA ligase. The catenated DNA was cloned into Hinc II digested pUC18 plasmid. E. coli DH5α (BRL) was transformed with the recombinant plasmid. A clone containing six Nla IV/Xho I fragments ligated in tandem was selected from the recombinants and designated as pAFE(Nla IV/Xho I)₆.

This plasmid was cleaved with restriction enzymes BamH I and Hind III. 3' termini of the obtained BamH I /Hind III fragment were labelled with DNA polymerase I Klenow fragment and 5'-[α³²P]dCTP (Amersham) and used as a probe for screening.

### ⑧ Method of screening (1):

Screening of the λ gt11 cDNA library prepared from cDNA which was synthesized by using random primer, was performed according to the known procedure [Cell, 52, 415, (1988)]. As a result, cDNA clone λ2 was isolated.

### ⑨ Method of screening (2):

Clones other than λ2 were isolatedby screening cDNA libraries with ³²P-labelled λ2 cDNA according to the method of Benton, W.D. and Davis, R. W. [Science 196, 180 (1977)].

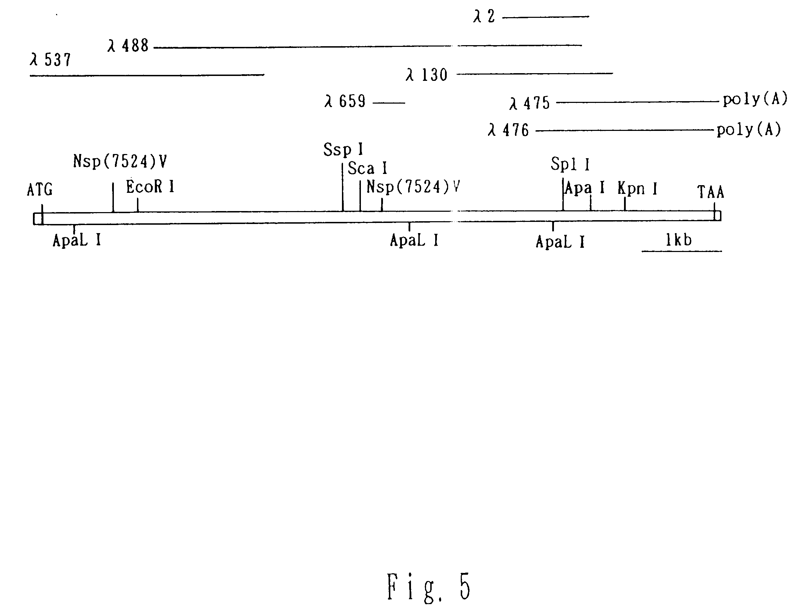

λ130, λ475, λ476 and λ488 were isolated by using λ2 cDNA as a probe. λ537 and λ659 were isolated by using portions of λ488 as probes. The positions of each cDNA clone are shown in Fig.5.

The fact that the protein encoded by the λ2 cDNA, recognizes TTAATAATT sequence was confirmed by gel shift assay and footprint analysis, as described below.

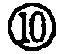 Preparation of lysogen extracts:

E.coli Y1089 was transfected with the recombinant phage according to a known method [DNA cloning, Vol. 1, p.49; Ed. by D.M. Grover, IRL Press(1985)] to produce lysogen . Extract was prepared according to Singh et al.[Cell, 52, 415 (1988)]. As a control, extract was also prepared from E. coli Y1089 which was transfected with λ gt11.

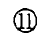 Gel Shift Assay:

Gel shift assay was performed according to the known method [Nature, 319, 154 (1988)] using the above mentioned lysogen extracts and DNA probe prepared from pAFE(HgiA I /BstN I)1 described in ⑦, as follows.

pAFE(HgiA I/BstN I)1 was digested with Sma I and Hinc II to prepare the enhancer DNA fragment. Probe was prepared by labelling the 5'-ends of the Sma I /Hinc II fragment with ³²P.

As shown in Fig.6, two shifted bands (a and b) were detected with the extract made from λ2 lysogen, while such bands were undetectable with λ gt11 lysogen extract. The result indicated that protein encoded by the cDNA cloned in λ2 has a property of binding to the probe DNA.

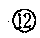 Identification of protein-binding site on the DNA fragment by footprint analysis:

λ2 lysogen extract containing 16 µg protein (as total protein) and Sma I/Hinc II probe, prepared from pAFE (HgiA I/BstN I)1 described in ⑦, were mixed in a 75 µl of reaction solution containing 10mM of Tris-HCl (pH 7.5), 50mM NaCl, one mM DTT, 0.5mM of EDTA, 2.5mM of MgCl₂, three µg poly (dI-dC) · poly(dI-dC) and 5% glycerol, and allowed to stand at room temperature for 30 minutes. The probe used in footprint analysis is labelledwith ³²P on only one of the 5' termini. To this mixture, one µl of DNase I (40 ng/µl) was added and the mixture was incubated for one minute at room temperature, after which 2.3 µl of 0.5M EDTA was added to cease the reaction. The reaction mixture was applied to a 5% polyacrylamide gel, and was subjected to electrophoresis at 11 V/cm until bromphenol blue migrated close to the lower end of the gel.

After removing one glass plate, the gel was wrapped with Saranwrap (Asahi Chemical Ind. Co., Ltd.) and autoradiographed.

The portion of gel corresponding to the position of DNA bound to protein and that of unbound DNA, determined by the relative mobility of the bands on the autoradiogram, was cut out of the gel. The gel section was immersed in 0.5ml solution composed of 0.5 M ammonium acetate, 0.1% of SDS and one mM of EDTA, and allowed to stand overnight at room temperature to extract the DNA. The mixture was centrifuged (12,000g, 10 min.) to obtain the supernatant. The gel section was washed twice with 0.5ml each of the same elution buffer and the wash was combined with the eluate. To this eluate, five µg of yeast transfer RNA (Sigma) was added and the resultant mixture was extracted with a mixture of phenol and chloroform (1:1). To the aqueous phase thus obtained, two volumes of ethanol was added and then the mixture was allowed to stand for 30 minutes or longer at -70°C. The DNA was then collected by centrifugation (12,000g, 10min.), washed once with 70% ethanol and was dried under vacuum. This DNA was dissolved in 10 µl solution composed of 80% formamide, 10mM of NaOH, one mM of EDTA, 0.1% xylenecyanol and 0.1% bromophenol blue, heated at 90°C for three minutes, and 3-4 µl of the solution was applied to an 8% polyacrylamide gel containing seven M urea, which is commonly used to determine nucleotide sequences. The samples were subjected to electrophoresis. Size markers prepared from the probe DNA by chemically cleaving at guanine and adenine residues [Methodsin Enzymology, 65, 499 (1980)] were run side by side with samples.

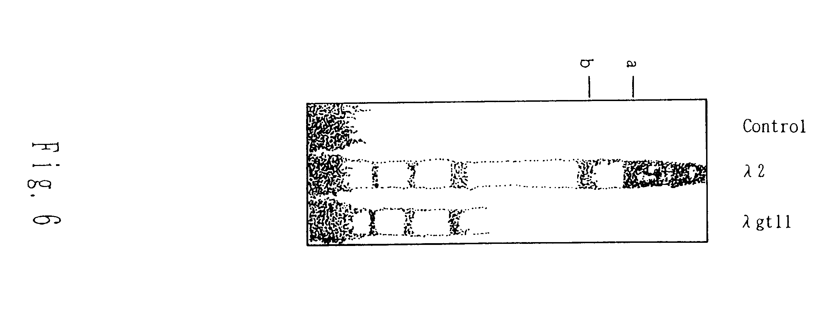

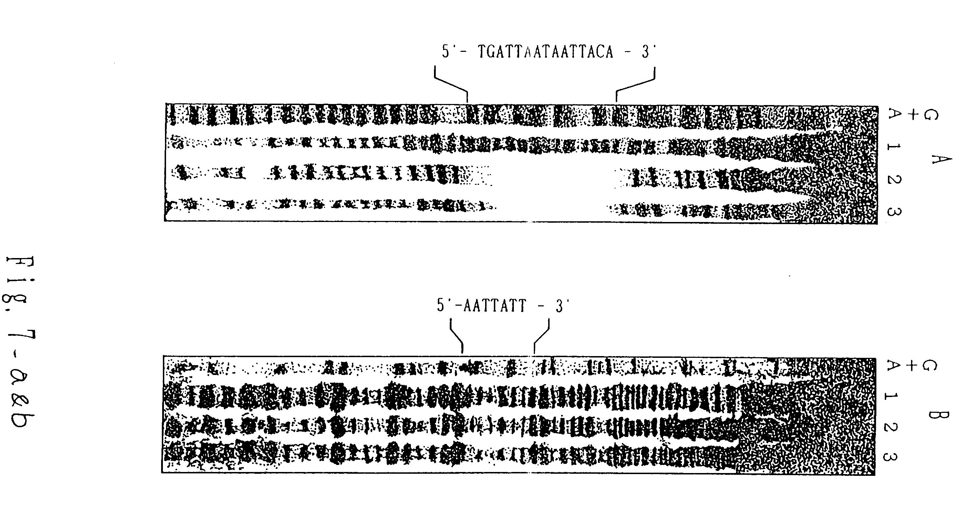

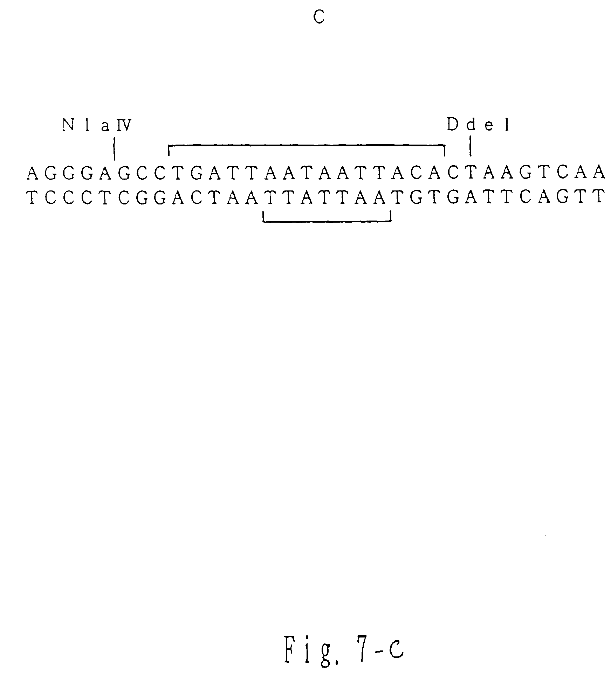

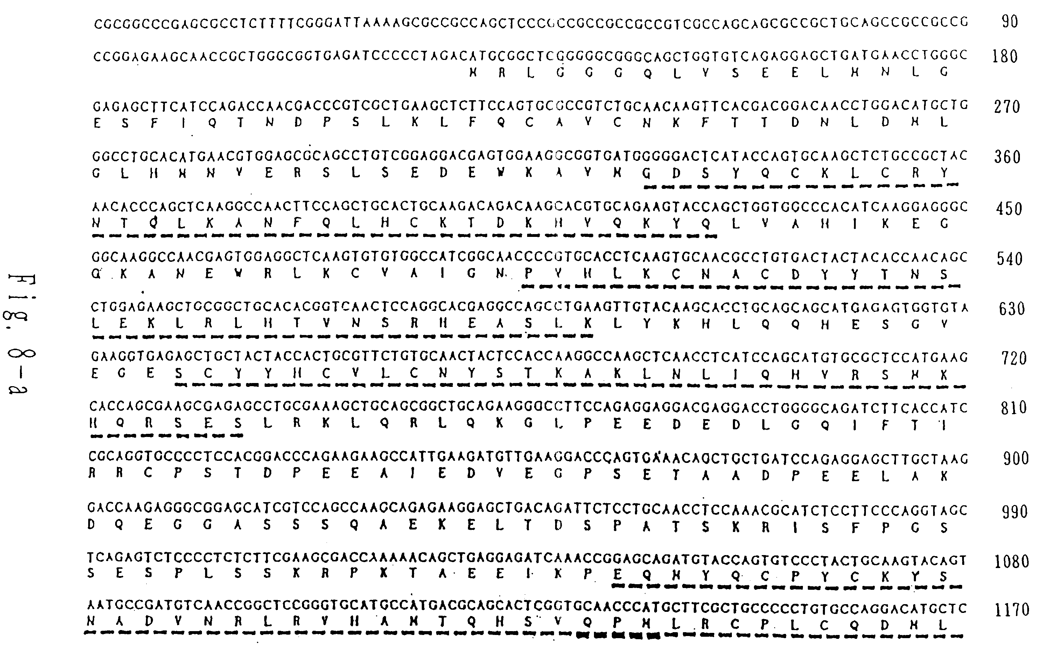

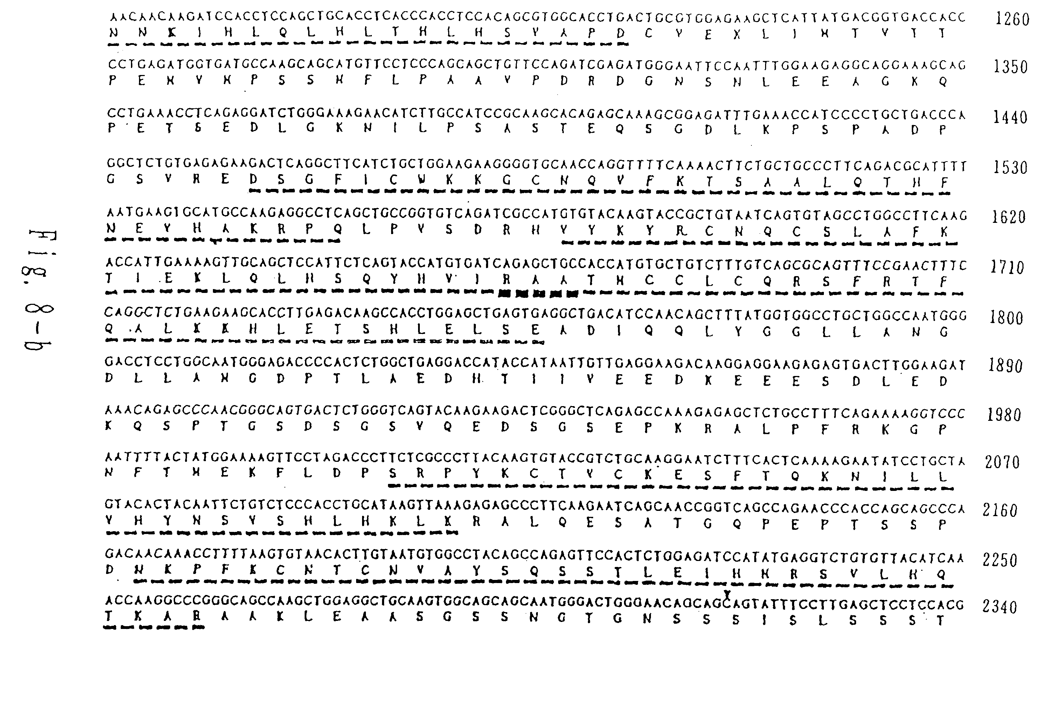

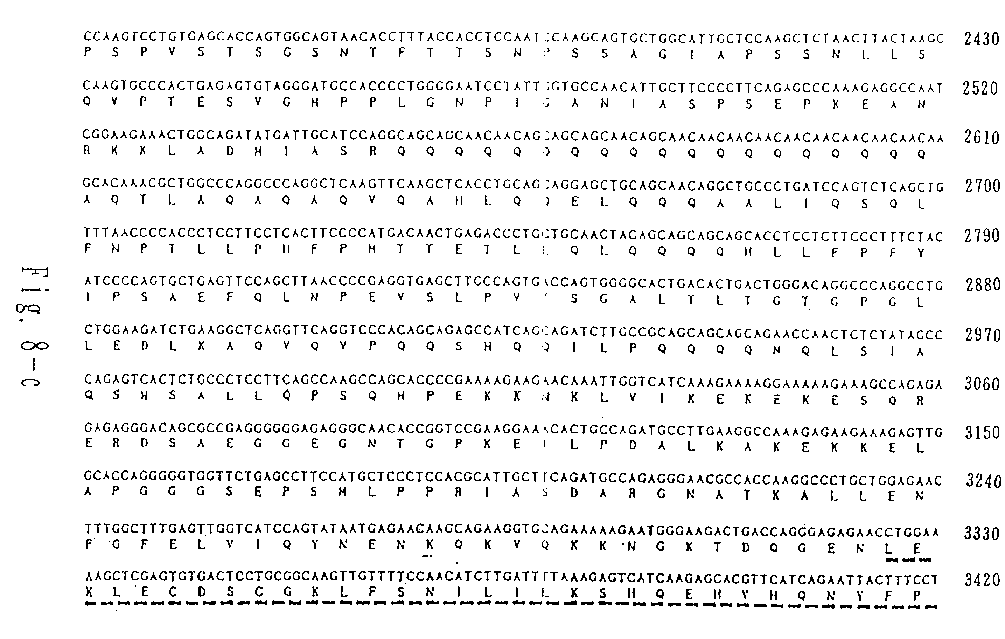

The result of autoradiography performed after electrophoresis are shown in Fig.7. As shown in the figure, it was indicated that, in DNA bound to the protein encoded by λ2 cDNA, the 15 base-pair portion containing TTAATAATTA in the midle was protected from cleavage by DNase I.

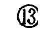 Nucleotide sequence of the cDNA

Phage DNA was prepared according to the conventional method [Molecular Cloning, a Laboratory Manual, p.76 (1982)]. After hydrolysis with EcoR I, insert DNA fragment was separated by agarose gel electrophoresis. The purified insert DNA fragment was subcloned in plasmid pUC118 or plasmid pBluescript II SK⁺ and amplified in E.coli DH5 α.

Fig.8 shows the nucleotide sequence of the cDNA, determined by dideoxy method [Method in Enzymology, 65, 560 (1980)] and Maxam-Gilbert method [Method in Enzymology, 65, 499 (1989)], and the amino acid sequence of the protein encoded by the cDNA.

Several nucleotide sequence heterogeneities were found in overlapping clones. At nucleotide 2316, λ 537 has cytosine (C), but λ488 has thymine (T).

At nucleotide 4338, λ659 has thymine (T), but λ 488 has cytosine (C). At nucleotide 5565, λ130 has thymine (T), but λ488 has cytosine (C). At nucleotide 7508, λ475 has cytosine (C), but λ476 has thymine(T). At nucleotide 8403, λ475 has guanine (G), but λ476 has thymine (T). Among these,the mutation at nucleotide 7508 resulted in the replaced amino acid residue (alanine residue in λ475 versus valine residue in λ476). Differences derived from deletion and insertion were also found in addition to the point mutations. An adenine(A) at nucleotide 4378 was deleted in λ488, however it was not deleted in λ659. Deletion of three consecutive nucleotides at two places, namely from nucleotide 7903 to 7905 and from 7921 to 7923, and insertion of 24 nucleotides (GG CAA CTA CAG CAG CAG CAG CAG C) between nucleotide 7528 and 7529 were observed in λ476 in comparison with λ475. These alterations resultedin an increase of six amino acid residues in the polypeptide encoded by λ476.

The nucleotide sequence shown in Fig.8 is based on λ475.

DNA encoding the full length enhancer binding protein was constructed by ligating the cDNAs described above and inserted to pBluescript II SK⁺. E. Coli harbouring the recombinant plasmids were deposited at the Fermentation Reseach Institute.

pBSAFP1A/DH5α (deposit number BP-3147) has the nucleotide sequence of λ476. pBSAFP1B/DH5α (deposit number BP-3148), on the other hand; has the nucleotide sequence of λ475. Both pBSAFP1A/DH5α and pBSAFP1B/DH5α have thymine, cytosine and cytosine at nucleotide 2316, 4338 and 5565, respectively.

## Claims (Claims for the following Contracting State(s): AT, BE, CH, LI, DE, DK, FR, GB, IT, LU, NL, SE)

1. A DNA which codes for a protein that has an amino acid sequence given in Fig. 8 and that binds specifically to the human α-fetoprotein gene enhancer.

2. A DNA which has a nucleotide sequence given in Fig. 8, in which a cytosine at nucleotide 7508 may be replaced by other nucleotides and, as a consequence, the amino acid 2460 (alanine) in the encoded protein may be replaced by other amino acids.

3. A DNA described in claim 2 in which a cytosine at nucleotide 7508 is replaced by a thymine.

## Claims (Claims for the following Contracting State(s): ES)

1. A DNA which codes for a protein that has an amino acid sequence given in Fig. 8 and that binds specifically to the human α-fetoprotein gene enhancer.

2. A DNA which has a nucleotide sequence given in Fig. 8, in which a cytosine at nucleotide 7508 may be replaced by other nucleotides and, as a consequence, the amino acid 2460 (alanine) in the encoded protein may be replaced by other amino acids.

3. A DNA described in claim 2 in which a cytosine at nucleotide 7508 is replaced by a thymine.

4. A method of producing a biologically active protein comprising inserting a DNA which codes for a protein that has an amino acid sequence given in Fig. 8 and that binds specifically to the human α-fetoprotein gene enhancer into an expression vector and co-transfecting animal cells with another expression vector in which a gene for the biologically active protein is placed under the control of α-fetoprotein gene enhancer and promoter.

5. A method according to claim 4 in which the cytosine at nucleotide 7508 may replaced by other nucleotides, preferably thymine, and the amino acid 2460 (alanine) in the encoded protein is replaced by other amino acids

## Patentansprüche (Patentansprüche für folgende(n) Vertragsstaat(en): AT, BE, CH, LI, DE, DK, FR, GB, IT, LU, NL, SE)

1. DNA, die ein Protein kodiert, das eine in Figur 8 angegebene Aminosäurensequenz aufweist und das spezifisch an den menschlichen α-Fetoprotein-Genverstärker bindet.

2. DNA, die eine in Figur 8 angegebene Nukleotidsequenz aufweist, worin ein Cytosin am Nukleotid 7508 durch andere Nukleotide ersetzt werden kann und als Konsequenz die Aminosäure 2460 (Alanin) in dem kodierten Protein durch andere Aminosäuren ersetzt werden kann.

3. DNA nach Anspruch 2, worin ein Cytosin am Nukleotid 7508 durch ein Thymin ersetzt wird.

## Patentansprüche (Patentansprüche für folgende(n) Vertragsstaat(en): ES)

1. DNA, die ein Protein kodiert, das eine in Figur 8 angegebene Aminosäurensequenz aufweist und das spezifisch an den menschlichen α-Fetoprotein-Genverstärker bindet.

2. DNA, die eine in Figur 8 angegebene Nukleotidsequenz aufweist, worin ein Cytosin am Nukleotid 7508 durch andere Nukleotide ersetzt werden kann und als Konsequenz die Aminosäure 2460 (Alanin) in dem kodierten Protein durch andere Aminosäuren ersetzt werden kann.

3. DNA nach Anspruch 2, worin ein Cytosin am Nukleotid 7508 durch ein Thymin ersetzt wird.

4. Verfahren zur Herstellung eines biologisch aktiven Proteins, das das Einfügen einer DNA, die ein Protein kodiert, welches eine in Figur 8 angegebene Aminosäurensequenz aufweist und spezifisch an den menschlichen α-Fetoprotein-Genverstärker bindet, in einen Expressionsvektor und das Co-Transfizieren von tierischen Zellen mit einem anderen Expressionsvektor umfaßt, in dem ein Gen für das biologisch aktive Protein unter die Kontrolle eines α-Fetoprotein-Genverstärkers und Promotors gebracht wird, umfaßt.

5. Verfahren nach Anspruch 4, worin das Cytosin am Nukleotid 7508 durch andere Nukleotide, vorzugsweise Thymin, ersetzt werden kann und die Aminosäure 2460 (Alanin) in dem kodierten Protein durch andere Aminosäuren ersetzt wird.

## Revendications (Revendications pour l'(les) Etat(s) contractant(s) suivant(s): AT, BE, CH, LI, DE, DK, FR, GB, IT, LU, NL, SE)

1. ADN qui code pour une protéine qui a la séquence d'acides aminés donnée dans la Figure 8, et qui se lie spécifiquement à l'amplificateur du gène de l'α-fétoprotéine humaine.

2. ADN qui a la séquence de nucléotides donnée dans la Figure 8, dans lequel une cytosine au nucléotide 7508 peut être remplacée par d'autres nucléotides et, en conséquence, l'acide aminé 2460 (alanine) dans la protéine encodée peut être remplacé par d'autres acides aminés.

3. ADN décrit dans la revendication 2, dans lequel une cytosine au nucléotide 7508 est remplacée par une thymine.

## Revendications (Revendications pour l'(les) Etat(s) contractant(s) suivant(s): ES)

1. ADN qui code pour une protéine qui a la séquence d'acides aminés donnée dans la Figure 8, et qui se lie spécifiquement à l'amplificateur du gène de l'α-fétoprotéine humaine.

2. ADN qui a la séquence nucléotidique donnée dans la Figure 8, dans lequel une cytosine au nucléotide 7508 peut être remplacée par d'autres nucléotides et, en conséquence, l'acide aminé 2460 (alanine) dans la protéine encodée peut être remplacé par d'autres acides aminés.

3. ADN décrit dans la revendication 2, dans lequel une cytosine au nucléotide 7508 est remplacée par une thymidine.

4. Procédé de production d'une protéine biologiquement active, dans lequel on insère un ADN qui code pour une protéine qui a la séquence d'acides aminés donnée dans la Figure 8, et qui se lie spécifiquement à l'amplificateur du gène de l'α-fétoprotéine humaine dans un vecteur d'expression, et on co-transfecte des cellules animales avec un autre vecteur d'expression dans lequel un gène pour la protéine biologiquement active est mis sous la commande de l'amplificateur et du promoteur du gène de l'α-fétoprotéine.

5. Procédé selon la revendication 4, dans lequel la cytosine au nucléotide 7508 peut être remplacée par d'autres nucléotides, de préférence la thymine, et l'acide aminé 2460 (alanine) dans la protéine encodée, est remplacé par d'autres acides aminés.
